# EUROPEAN PATENT SPECIFICATION

(11) **EP 3 470 111 B1**
(45) Date of publication and mention of the grant of the patent: **31.12.2025**
(21) Application number: 18200222.0
(22) Date of filing: 12.10.2018
(51) Int. Cl.: A61N 1/36, A61B 5/01, A61B 5/00, A61B 5/145

(54) **VAGUS NERVE STIMULATION TO TREAT NEURODEGENERATIVE DISORDERS**
NERVUS VAGUS-STIMULATION ZUR BEHANDLUNG VON NEURODEGENERATIVEN ERKRANKUNGEN
STIMULATION DU NERF VAGUE POUR TRAITER DES TROUBLES NEURODÉGÉNÉRATIFS

(30) Priority: 13.10.2017 US 201762572374 P; 24.10.2017 US 201762576547 P; 11.10.2018 US 201816158222
(43) Date of publication of application: 17.04.2019
(73) Proprietor: Setpoint Medical Corporation, Valencia, CA 91355-5004 (US)
(72) Inventor: HAMLIN, Nicole, Valencia CA 91355-5004 (US); LEVINE, Jacob A., Valencia CA 91355-5004 (US); CHERNOFF, David, Valencia CA 91355-5004 (US)
(74) Representative: Potter Clarkson

(56) References cited:
- US-A1- 2008 125 819
- US-A1- 2009 062 874
- US-A1- 2009 312 817
- US-A1- 2012 290 035
- US-A1- 2013 253 413
- US-A1- 2014 046 407
- US-A1- 2014 288 551
- US-A1- 2016 096 016
- US-B1- 6 762 032
- HOUSLEY WILLIAM J ET AL: "Biomarkers in multiple sclerosis", CLINICAL IMMUNOLOGY, ELSEVIER, AMSTERDAM, NL, vol. 161, no. 1, 2 July 2015 (2015-07-02), pages 51 - 58, XP029321509, ISSN: 1521-6616, DOI: 10.1016/J.CLIM.2015.06.015

## Description

### FIELD

The present disclosure relates generally to apparatuses (e.g., devices, systems) for vagus nerve stimulation to treat neurodegenerative and neuroinflammatory disorders, and more specifically apparatuses for vagus nerve stimulation to reduce demyelination and/or to promote remyelination to treat various neurodegenerative disorders such as multiple sclerosis.

### BACKGROUND

A variety of central nervous system (CNS) demyelinating disorders, including multiple sclerosis, acute disseminated encephalomyelitis and neuromyelitis optica spectrum disorders, are difficult to effectively treat. For example, multiple sclerosis (MS) is a neurodegenerative disease characterized by demyelination of nerves in the central nervous system. Although the root cause of demyelination is not well understood, it generally is associated with the formation of lesions on the myelin sheaths and inflammation. Currently, there is no known cure for MS. Current treatments, with modest success, are primarily directed to treating acute attacks and reducing the frequency of attacks in the relapsing-remitting subtype of the disease or treating the symptoms. However, current therapies at best only slow the progression of the disease, and no therapy to date has demonstrated an ability to remyelinate nerves.

Therefore, it would be desirable to provide additional treatment systems that can be used independently or in conjunction with other therapies to reduce the rate or amount of demyelination. Furthermore, it would desirable to provide a therapy that remyelinates nerves and reverses the progression demyelination. In addition, it would be desirable to reduce inflammation in the nervous system.

US 2016/096016 discloses a system and method for treating inflammation by vagus nerve stimulation and the delivery of an anti-inflammatory drug.

US 2012/0290035 discloses a system and method for applying extremely low duty-cycle stimulation for treating chronic inflammation. The system includes an implantable microstimulator and a controller for setting a dose for the microstimulator.

US 2014/0288551 discloses a system and method for treating human anemia. An external neurostimulation system is described as well as a chronic implantable system.

US 2014/046407 discloses a nerve stimulation system comprising an electrode device that is configured to be coupled to a parasympathetic site of a patient and a controller for driving the electrode device to apply a current in bursts of one or more pulses.

US 2009/062874 A1 shows inhibition of granulocyte activation by appropriate stimulation of the vagus nerve, whereby the level of granulocyte activation may be detected and used to at least partially control stimulation.

US 6 762 032 B1 shows the link between multiple sclerosis and demyelination.

### SUMMARY OF THE DISCLOSURE

According to the present invention there is provided a system for reducing demyelination and/or increasing remyelination by stimulation of a vagus nerve according to claim 1. The present invention is defined by the appended claims.

Methods described hereinafter are not claimed and only of exemplary nature, and presented to better understand the present invention.

The present disclosure relates generally to vagus nerve stimulation to treat neurodegenerative disorders, and more specifically to vagus nerve stimulation to reduce demyelination and/or to promote remyelination to treat various neurodegenerative disorders such as multiple sclerosis.

Described herein are apparatuses (e.g., devices and/or systems) for reducing demyelination and/or increase remyelination by stimulation of a vagus nerve. These apparatuses may be implants or implanted into the patient's body. The apparatuses include: a biosensor configured to detect one or more biomarkers; a stimulator configured to apply stimulation to the vagus nerve; and a controller coupled to the biosensor and the stimulator and configured to apply stimulation to the vagus nerve from the stimulator sufficient to reduce demyelination and/or increase remyelination of nerves within the patient when the biosensor detects a biomarker indicative of demyelination. In some variations, these apparatuses include an implant comprising a stimulator (e.g., a waveform and/or pulse generator, an oscillator, a power supply and/or power regulation circuit, etc.), a stimulation applicator (e.g., one or more electrodes, mechanical transducers, etc.), and a controller. The controller may be configured as a microcontroller and may be in electrical communication with the stimulator so as to control operation of the stimulator. The controller may include one or more processors, a memory and/or a timer. The stimulator and/or controller may be in electrical communication, one or more stimulation applicators. In some variations the controller may include or be in communication with wireless communications circuitry for wirelessly communicating with one or more remote processors. The remote processor may be a hand-held device (e.g., smartphone, wearable electronics, etc.). The controller may optionally be in communication with one or more biosensors that may be included with the implant or may be remote from the implant (e.g., may be wearable, single-use, etc.). In some variations the biosensors are wirelessly connected to the apparatus.

In some variations, not claimed, the apparatus may be used without a biosensor. For example, the apparatus may be configured to periodically and/or on demand apply VNS treatment to prevent or reduce demyelination. The apparatus may be configured to apply VNS treatment doses once multiple times per day (e.g., 1x day, 2x, day, 3x, day, 4x day, 5x day, 6x day), or every other day, or every 3 days, etc. In some variations the apparatus may be configured to both automatically apply a VNS treatment dose on a predetermined and/or adjustable scheduled, as well as provide VNS treatment doses based on input from a user (e.g., patient, physician, etc., including "on demand" doses) and/or based on detection of a biomarker indicative of an actual or potential increase in demyelination.

In any of these variations, a biosensor may be configured to detect one or more markers (e.g., biomarkers) from the patient's body, including from the patient's blood and/or cerebrospinal fluid. Examples of biomarkers may be found herein. The biosensor may be part of the implanted apparatus, or it may be connected to the apparatus (e.g., the controller) via a wired or wireless communication. The biosensor may be configured to detect any biological marker, including chemical markers (e.g., a protein, nucleotide, e.g., RNA, DNA, microRNA, etc., lipid, carbohydrate, etc.), as well as functional markers (nerve conduction, etc.), body temperature, and the like. For example, in some variations, the biosensor is configured to detect temperature.

In general, the apparatuses described herein may be configured to be inserted or implanted into the body. For example, the apparatus may be configured to be implanted. The apparatus may include a stimulation applicator (also referred to as simply a stimulator or a VNS treatment stimulator) that may be a mechanical and/or electrical stimulator. A mechanical stimulator may be a piezoelectric driver that may vibrate and/or apply pressure to the tissue, including to the vagus nerve, in the VNS treatment parameters, such as mechanical stimulation of the vagus nerve at between 1-2 kHz for a treatment time (e.g., between 1 ms and 5 minutes, e.g., 10 ms-10 sec, etc.). Alternatively or additionally, the stimulation applicator may be an electrical stimulation applicator and may include one or more (e.g., two or more) electrodes configured to apply electrical stimulation to the vagus nerve. For example, electrical stimulation of about 0.1 mA to 10 mA (e.g., between 1 mA - 5 mA), at a frequency of between about 1Hz and about 2 kHz (e.g., between about 1 - 100 Hz), where the pulses applied have a pulse width of between about (50-500 usec, e.g., between about 100-300 usec). The controller may be configured to enforce an 'off-time' following a VNS treatment dose of between about 10 minute and 12 hours (e.g., between about 2 hours and 10 hours, between about 3 hours and 6 hours, at least 2 hours, at least 3 hours, at least 4 hours, at least 5 hours, at least 6 hours, etc.). For example, the stimulator may include an electrode configured to apply electrical energy to the vagus nerve.

In some variation the apparatus is configured to apply VNS treatment to the patient in which the VNS treatment is electrical stimulation. For example, the VNS treatment may include the application of electrical energy at between about 1-100 Hz (e.g., between about 1-50 Hz, between about 1-20 Hz, between about 5-30 Hz, between about 5-15 Hz, approximately 5 Hz, approximately 10 Hz, approximately 15 Hz, etc.). The energy may have a peak amplitude of between about 0.1 mA and about 2 mA (e.g., between about 0.2 mA and about 1.8 mA, between about 0.5 mA and about 1.5 mA, between about 0.5 mA and about 1 mA, between about 0.1 mA and about 1 mA, approximately 0.5 mA, approximately 0.75 mA, approximately 1 mA, etc.). Alternatively the applied energy may have an average amplitude of between about 0.1 mA and about 2 mA (e.g., between about 0.2 mA and about 1.8 mA, between about 0.5 mA and about 1.5 mA, between about 0.5 mA and about 1 mA, between about 0.1 mA and about 1 mA, approximately 0.5 mA, approximately 0.75 mA, approximately 1 mA, etc.). The applied energy is typically pulsed, and may be pulsed square waves, sinusoidal waves, triangular waves, etc. The applied energy may be biphasic or monophasic. For example, the applied energy maybe biphasic. The applied VNS treatment may be a constant biphasic pulse train having a frequency of between 1-100 Hz (e.g., 10 Hz) and a peak amplitude of between about 0.5 mA and 2 mA (e.g., approximately 0.75 mA). Any of the methods for treatment described herein may be configured to apply this type of VNS treatment.

Any of the apparatuses (e.g., devices, systems, etc.) described herein may be configured to be implanted on the vagus nerve. Thus, any of these apparatuses may be implanted via a nerve sheath or nerve cuff configured to secure the apparatus onto the nerve and/or prevent movement of the apparatus relative to the nerve and/or insulate the apparatus from other tissues. The implanted apparatus may be implanted in any appropriate location on the nerve, including one or around the vagus nerve at the upper chest, or on or around the vagus nerve at a sub-diaphragmatic location. The implant may be a leadless implant that is connected to the vagus (see, e.g., US 8412338, US 8612002, US 8886339, and US 8788034). For example, any of these apparatuses may include a nerve cuff configured to secure the stimulator to the vagus nerve. Alternatively, any of these apparatuses may include a lead connecting the micro stimulator and/or other components to the stimulation applicator on/around the vagus nerve via one or more leads.

As mentioned, any of these apparatuses may be configured to apply VNS treatment comprising a low duty-cycle electrical stimulation of between about 0.25 mA and about 5 mA to the vagus nerve for less than about 2 minutes. The apparatus may be configured to provide an off-time of at least x minutes/hours (e.g., 10 minutes, 20 minutes, 30 minutes, 40 minutes, 60 minutes, 2 hours, 3 hours, 4 hours, etc.).

Any of the apparatuses described herein may be configured to perform a method of reducing demyelination in a patient diagnosed with or at risk of a disorder involving demyelinated nerves (e.g., including but not limited to methods of treating a disorder and/or disease associated with demyelination, such as multiple sclerosis). For example, a method of reducing demyelination (and/or a method of increasing remyelination) may be a method comprising detecting a marker for demyelination and applying stimulation to the vagus nerve to reduce demyelination of nerves within the patent.

Applying stimulation to the vagus nerve includes applying VNS treatment and may comprise, for example, applying electrical stimulation of between about 0.25 and about 5 mA to the vagus nerve for less than about 2 minutes. In some variations this may include waiting for an off-time (e.g., an off-time of at least 10 minutes, 30 minutes, 1 hour, 2 hours, 3 hours, 4 hours, etc.).

Any of these methods may include applying non-invasive stimulation to the vagus nerve. For example, the simulation may be through a transdermal (e.g., via a surface electrode and/or mechanical stimulation, including ultrasound) route over a portion of the vagus nerve. The vagus nerve includes a number of branches or extensions that may be accessed and/or targeted from outside of the body either mechanically and/or electrically. For example, non-invasive application may include ultrasound stimulation of the vagus nerve. Any of these methods may include applying transdermal electrical stimulation (TENS), or the like.

Any of the methods described herein may include monitoring, e.g., periodically, on demand, and/or continuously, one or more markers (e.g., biomarkers) for demyelination or a risk of demyelination. As mentioned, any appropriate method or apparatus for monitoring demyelination or a risk of demyelination may be used. For example any of these methods may include detecting a marker for demyelination comprising monitoring the patient's temperature. A change (including an increase) in core body temperature has been linked to an increase in symptoms in demyelination disorders, including but not limited to MS.

Any of the methods and apparatuses described herein may be used with or linked to markers for the integrity of the blood-brain barrier. The methods and apparatuses described herein generally improve the integrity of the blood-brain barrier. Thus, any marker linked to leakage or loss of integrity of the blood-brain barrier may be used to trigger VNS therapy as described herein. Examples of markers may include Serum S100β, as well as imaging modalities such as contrast-enhanced magnetic resonance imaging, CT-scan and lumbar puncture.

A detection of one or more markers (e.g., biomarkers) for demyelination may include determining a level of tumor necrosis factor in a blood or cerebrospinal fluid sample.

For example, described herein are exemplary methods (e.g., methods of treating a demyelination disorder, such as but not limited to MS, and/or methods of reducing or reversing demyelination) that include: detecting demyelination in a patient, and applying stimulation to the vagus nerve to increase the remyelination of nerves within the patent.

For example, any of these methods may include repeatedly applying a low duty-cycle electrical stimulation of between about 0.25 and about 5 mA to the patient's vagus nerve for less than about 2 minutes, followed by an off-time (e.g., of between about 10 minutes and about 48 hours) before the next stimulation.

Any of these methods and apparatuses may also include or be adapted to include the concurrent (immediately before, during or after, including systemically and/or locally) treatment with one or more pharmacological agents, particularly those that are believed to help with a demyelinating condition, such as (but not limited to) MS. For example, any of these method may include concurrently treating with a pharmacological agent such as one or more of: interferon beta-1a, interferon beta-1b, glatiramer acetate, glatiramer acetate, peginterferon beta-la, daclizumab, teriflunomide, fingolimod, dimethyl fumarate, alemtuzumab, mitoxantrone, ocrelizumab, natalizumab.

As mentioned, any of the methods and apparatuses described herein may include continuously monitoring the patient for demyelination or a condition implicated in demyelination. For example, any of these methods and apparatuses described herein may include monitoring the patient for a marker related to a diseased selected from the group consisting of neurodegenerative diseases, neuroinflammatory diseases, and neuropathies. In some examples, the method includes detecting demyelination in a patient by detecting a marker related to MS. For example, the marker (e.g., biomarker) may be selected from the group including: neurofilament, glial fibrillary acidic protein, the monocyte macrophage marker CD163, the glial activation marker YKL-40, the B cell chemoattractant CXCL13, miRNA, mRNA, myelin reactive t cells, Kir4.1 antibodies, osteopontin, and microbiome associated lipopeptides.

### BRIEF DESCRIPTION OF THE DRAWINGS

The features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings of which:
FIG. 1 illustrates a typical example of the 4 epochs that follow lysolecithin injection to the spinal cord in a model used to study multiple sclerosis.
FIGS. 2A and 2B illustrate the experimental protocols used to study demyelination and remyelination.
FIG. 3A illustrates a cross-section of a healthy spinal cord.
FIG. 3B illustrates a stained cross-section of a spinal cord with a lesion (which may be considered a demyelination) induced by lysolecithin injection.

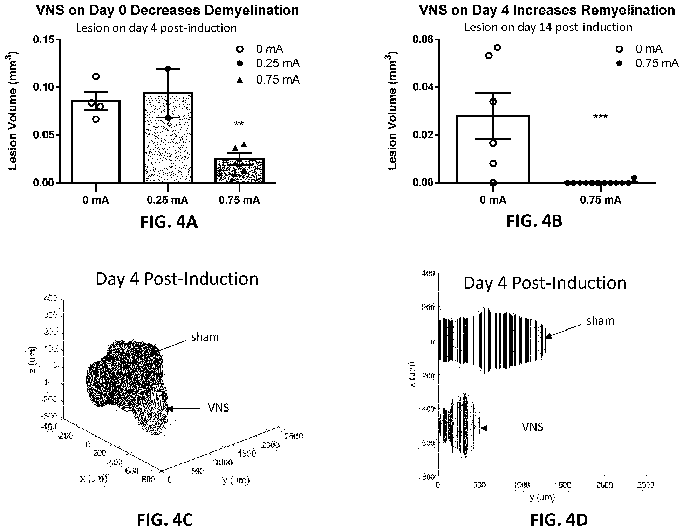

FIGS. 4A-4D are graphs that show that vagus nerve stimulation reduced the amount of demyelination that resulted from lysolecithin injection. FIG. 4A is a graph showing the effect of vagal nerve stimulation (VNS) on demyelination with various levels of stimulation (O mA, 0.25 mA, and 0.75 mA) four days following inducing of a demyelinating lesion. FIG. B shows the increase in remyelination by two weeks after inducing the demyelinating lesion without VNS treatment (0 mA) and with VNS treatment (0.75 mA), showing a rapid remyelination when VNS is applied. FIG. 4C is a 3D graph of the lesion size variation by depth at four days post induction of the demyelinating lesion with and without VNS treatment. FIG. 4D is a 2D projection graph of the median lesion four days post-induction of the demyelinating lesion comparing sham (no VNS treatment) and VNS treatment.

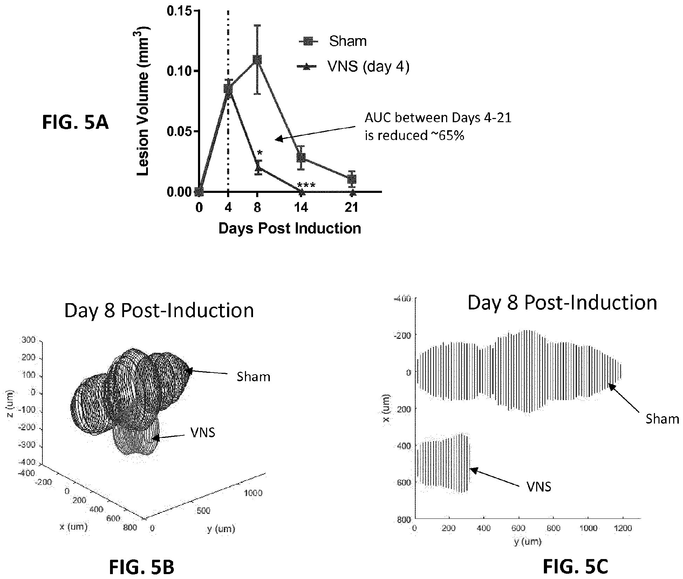

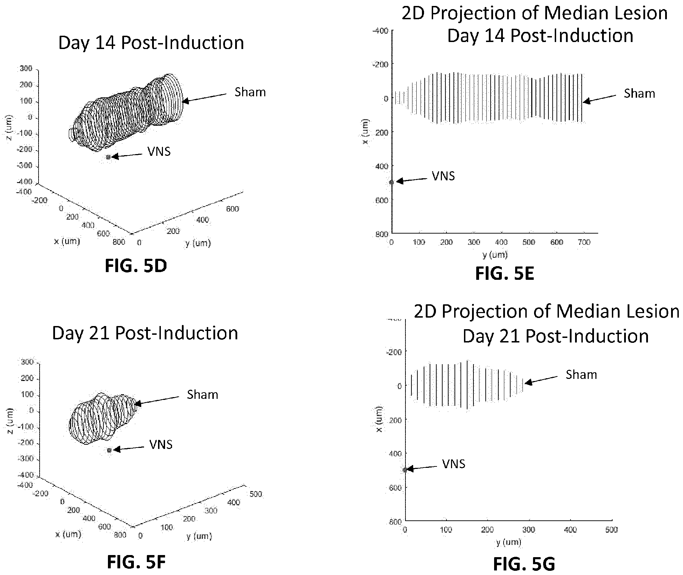

FIGS. 5A-5G are graphs that show that vagus nerve stimulation increased the rate and/or amount of remyelination. FIG. 5A is a graph showing the change in demyelination (determined by the change in induced lesion volume) following induction of demyelination with and without VNS treatment, showing an approximately 65% reduction in the area under the lesion volume (mm³)/days post induction. FIG. 5B is a 3D representation of the demyelination (lesion) size variation with depth for no VNS treatment (sham) and VNS treatment. FIG. 5C is a 2D projection of median lesion volume eight days post-induction of demyelination (e.g., lesion) with VNS treatment and without VNS treatment (sham). FIG. 5D is a 3D representation of demyelination (lesion size) variation with depth at day 14 following inducing of demyelination (day 14 post induction) with VNS treatment (VNS) and without VNS treatment (sham). FIG. 5E is a 2D projection of median demyelination (lesion) at two weeks post-induction of demyelination with VNS treatment and without VNS treatment ("sham"). FIG. 5F is a 3D representation of demyelination (lesion size) variation with depth at day 21 following inducing of demyelination (day 14 post induction) with VNS treatment (VNS) and without VNS treatment (sham). FIG. 5G is a 2D projection of median demyelination (lesion) at three weeks post-induction of demyelination with VNS treatment and without VNS treatment ("sham").
FIG. 6A shows the experimental protocol used to show the effect of VNS treatment as described herein on vessel leakiness following post-induction of demyelination.

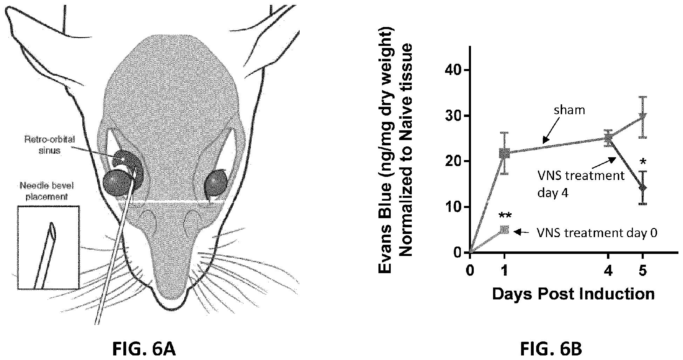

FIG. 6B illustrates the use of VNS treatment as described herein to reduce the leakiness of the blood-brain barrier following induced demyelination. VNS treatment before induced demyelination prevented the passage of dye (Evans blue) through the rat model of the blood brain barrier. VNS treatment after induced demyelination reduced and reversed the leakiness. VNS treatment on Day 0 (following LPC induction) significantly decreased leukocyte infiltration 24 hours post-stimulation, while VNS treatment on Day 4 post-LPC induction significantly decreases leukocyte infiltration 24 hours post-stimulation.
FIG. 7A illustrates the effect of the alpha-7 nicotinic acetylcholine receptors (α7 nAChR) in preventing demyelination re-myelination from VNS treatment (compared to sham without VNS treatment).
FIG. 7B illustrates the effect of the alpha-7 nicotinic acetylcholine receptors in increasing re-myelination from VNS treatment (compared to sham without VNS treatment).
FIG. 8 shows the effect of VNS treatment as described herein to prevent or reverse leakiness of the blood-brain barrier compared to sham (no VNS treatment). In FIG. 9, CD3+ T cell infiltration was significantly decreased in the VNS group on Day 3 post-LPC induction compared to Sham group by 50%.
FIG. 9 illustrates macrophage infiltration through a model of the blood-brain barrier is significantly decreased 24 hours post-demyelination induction (e.g., via LPC) with VNS treatment compared to sham (no VNS treatment) by 55%.
FIGS. 10A and 10B illustrate the effect of pro-resolution lipid Resolvin D1 following induction of demyelination with VNS treatment (VNS) and without VNS treatment (sham), showing Resolvin D1 (RvD1) is increased in VNS animals 4 days post-LPC induction compared to Sham animals and remains elevated 14 days post-LPC induction. Levels were decreased below that of the Sham 21 days post-LPC induction, by which time, no visible lesion is detected in VNS animals.
FIG. 11 schematically illustrates one example of an apparatus for reducing demyelination (e.g., increasing remyelination and/or reducing leakage through the blood-brain barrier), as described herein.

### DETAILED DESCRIPTION

Electrical and/or mechanical stimulation of the cholinergic anti-inflammatory pathway (NCAP) by stimulation of the carotid vagus nerve been well described. For example, see U.S. 6,838,471, U.S. 8,914,114, U.S. 9,211,409, U.S. 6,610,713, U.S. 8,412,338, U.S. 8,996,116, U.S. 8,612,002, U.S. 9,162,064, U.S. 8,855,767, U.S. 8,886,339, U.S. 9,174,041, U.S. 8,788,034 and U.S. 9,211,410. It has not previously been suggested that vagus nerve stimulation may be used to prevent or reduce demyelination and/or improve remyelination. Vagus nerve stimulation, through activation of both efferent and afferent pathways (or primarily through one of the efferent or afferent pathway), may be able to reduce the inflammation associated with inflammatory diseases and disorders, thereby reducing the severity of the symptoms and/or slowing, stopping, or reversing the progression of the disease. Interesting, the Applicants have surprisingly found that the apparatuses (e.g., systems, devices, etc.) and methods described herein may be used to stimulate the vagus nerve to reduce demyelination and/or to increase or promote remyelination. Furthermore, although the use of VNS treatment to modulate inflammation has been thought to involve afferent pathways, remyelination and demyelination may involve the efferent pathway or both the afferent and efferent pathways.

Diseases (e.g., diseases and disorder of myelination) which may benefit from VNS include, but are not limited to, multiple sclerosis (MS), Alzheimer's disease, Parkinson's disease, Huntington's disease, Amyotrophic lateral sclerosis (ALS), chronic inflammatory demyelinating polyneuropathy (CIDP), and Batten disease. Neuropathies that may benefit from VNS include peripheral neuropathies, cranial neuropathies, and autonomic neuropathies.

### Vagus Nerve Stimulation Systems and Devices

In some variations the devices described herein are electrical stimulation devices that may be implanted, and may be activated to apply current for a proscribed duration, followed by a period without stimulation. As described in the examples that follow, the stimulation protocol may comprise a very limited period of stimulation (e.g., an on-time of less than 5 minutes, 2 minutes, 1 minute, etc.) followed by an off-time (during which stimulation is not applied, and may be prevented from being applied) of extensive duration (e.g., greater than 10 minutes, 20 minutes, 30 minutes, 40 minutes, 50 minutes, 1 hour, 1.5 hours, 2 hours, 4 hours, 12 hours, greater than 20 hours, greater than 24 hours, greater than 36 hours, greater than 48 hours, etc.). The applied energy may be electrical energy that is a fixed current having a frequency that is within the range of about 0.5 mA to 5 mA (e.g., approximately 2 mA), at a frequency of between about 1Hz and about 1000 Hz (e.g., between 1 Hz and 100 Hz, between 1 Hz and 30 Hz, between 10 Hz and 200 Hz, etc.), where the pulses applied have a pulse width of approximately (50-500 usec, e.g., a 200 usec pulse). Thus, the duty-cycle of the applied current may be extremely low, where duty cycle may refer to the ratio of on-time/(on-time plus off-time). The stimulation is applied at an extremely low duty cycle, where duty cycle may refer to the percent of on-time to the total on-time and off-time for the ongoing treatment. The low duty cycle is less than about 10 percent and may, for example, be less than 5, 4, 3, 2, 1, or 0.5 percent of on-time to the total on time and off-time. The effect may be seen relatively quickly, and may persist over the entire off-time.

In particular, the methods and apparatuses described herein may be applied as needed, e.g., when the patient expresses or is likely to express an increased risk for demyelination and/or is experiencing (or has experienced) demyelination. Alternatively or additionally, the methods an apparatuses may be applied as needed when the patient expresses or is likely to express, and/or is experiencing (or has experienced) a leakage through the blood-brain barrier.

For example, we show herein that a low level, low duty cycle stimulation protocol (as described herein) reduces demyelination and/or increases remyelination, and prevents and/or reduces leakage through the blood-brain barrier. The effectiveness of low level, low duty cycle vagus nerve stimulation (VNS therapy) administered on even a single day results in a reduction in demyelination and an increase in remyelination seen over the course of two to three weeks. This type of stimulation contrasts with the use of a high duty cycle stimulation used by others to modulate vagus-nerve mediated functions (such as heart rate, etc.), or treat disorders such as epilepsy and depression. An important finding here is that demyelination can be reduced and even more surprisingly, remyelination can be increased. This effect is corroborated at these low duty cycle parameters by examining the histology of the spinal cord as described later below. Although low duty cycle vagus nerve stimulation is effective and highly efficient at reducing inflammation, in some embodiments, a higher duty cycle stimulation can be used, such as a duty cycle that is greater than about 1, 2, 3, 4, 5, 10, 20, 30, 40, or 50 percent of on-time to the total on-time and off-time.

MS patients may experience circadian pattern disruptions to symptoms that may be associated with or caused in part by the circadian patterns of IL-6 levels. Optionally, drugs, such as steroids, can be used along with VNS to suppress nighttime spiking of IL-6. Similarly, VNS can be modulated, by altering the timing of the stimulations for example, to suppress nighttime spiking of IL-6 more effectively. However, one advantage of VNS is the relatively long duration of the effect after a single stimulation, which may allow suppression of IL-6 levels during both night and day, which may render unnecessary the need for supplementary drug treatment or alternative timings. In some embodiments, VNS can be given in the evening before sleep, such as 15, 30, 45, 60, 90, 120, 150, or 180 minutes before sleep, and may also be given at night during sleep, to ensure nighttime suppression of IL-6 levels. In some embodiments, the amplitude of stimulation during sleep can be lowered (e.g., less than 2, 1.5, or 1 mA) to avoid waking the patient. In some embodiments, IL-6 levels can be measured and/or monitored, and VNS can be modulated based on the measured and/or monitored IL-6 levels. Other cytokines may also be measured and/or monitored, such as IL-1, TNF, IFN-gamma, IL-12, IL-18, and GM-CSF. These other cytokines may be used instead of or in addition to IL-6, either in combination or singly.

The methods, devices and systems herein may be applied specifically to treat any disorder for which a reduction of demyelination and/or an increase in remyelination would be beneficial. For example, described herein are electrodes (e.g., cuff electrodes, microstimulators) that may be placed around the vagus nerve and may communicate with one or more stimulators configured to apply appropriate stimulation of the vagus nerve to modulate demyelination and/or remyelination. The stimulator may be implanted. In some variations the stimulator is integral to the electrodes, and may be charged externally. The extremely low duty-cycle of the technique described herein may allow the device to be miniaturized to a greater degree than previously suspected for the treatment of chronic disorders via an implantable device.

In general, a device or system for modulating demyelination and/or remyelination may include a stimulator element (e.g., an electrode, actuator, etc.) and a controller for controlling the application of stimulation by the stimulator element. A stimulator element may be configured for electrical stimulation (e.g., an electrode such as a cuff electrode, needle electrode, paddle electrode, non-contact electrode, array or plurality of electrodes, etc.), mechanical stimulation (e.g., a mechanical actuator, such as a piezoelectric actuator or the like), ultrasonic actuator, thermal actuator, or the like. In some variations the systems and/or devices are implantable. In some variations the systems and/or device are non-invasive. In general, the controller may include control logic (hardware, software, firmware, or the like) to control the activation and/or intensity of the stimulator element. The controller may control the timing (e.g., on-time, off-time, stimulation duration, stimulation frequency, etc.). In variations in which the applied energy is electrical, the controller may control the applied waveform (amplitude, frequency, burst duration/inter-burst duration, etc.). Other components may also be include as part of any of these device or system, such as a power supply (e.g., battery, inductive, capacitor, etc.), transmit/receive elements (e.g., antenna, encoder/decoder, etc.), signal generator (e.g., for conditioning or forming the applied signal waveform), and the like. In some embodiments, a rechargeable battery that may be inductively charged allows the stimulator to deliver numerous electrical stimulations before needing to be recharged. In other embodiments, one or more capacitors that can also be inductively charged can be used to store a limited amount of energy that may be sufficient to deliver a single stimulation or a daily amount of stimulations. This dramatically reduces the size and cost of the stimulator, but requires that the user charge the stimulator daily or before each use.

In one example, an implantable device for modulating demyelination and/or remyelination (and/or reducing or preventing leaking of the blood-brain barrier) includes an electrode for electrically stimulating the vagus nerve. The electrode may be, for example, a cuff electrode. The electrode may be connected (directly or via a connector) to a controller and signal generator. The signal generator may be configured to provide an electrical signal to the electrode(s). For example, the electrical signal may be an electrical waveform having a frequency of between about 0.1Hz and about 1 KHz (e.g., 10 Hz), where the pulses applied have a pulse width of approximately (50-500 usec, e.g., a 200 usec pulse). The signal generator may be battery (and/or inductively) powered, and the electrical signal may be amplitude and/or voltage controlled. For example in some variations the device or system may be configured to apply a current that is between about 0.05mA to 25mA (e.g., approximately 0.5 mA, 1 mA, 2 mA, 3 mA, etc.). The electrical signal may be sinusoidal, square, random, or the like, and may be charge balanced. In general, the controller (which may be embodied in a microcontroller such as a programed ASIC), may regulate turning on and off the stimulation. For example, stimulation may be applied for an on-time of between about 0.1 sec and 10 minutes (e.g., between 1 sec and 5 minutes, between 1 sec and 2 minutes, approximately 1 minute, etc.); the stimulation may be configured to repeat automatically once every x hours or days, e.g., every other day (off time of approximately 48 hours), once a day (e.g., with an off-time of approximately 24 hours), twice a day (off-time of approximately 12 hours), three times a day (off time of approximately 8 hours), four times a day (off time of approximately 6 hours), or the like. In some variations the implant may be configured to receive control information from a communications device. The communications device may allow modification of the stimulation parameters (including off-time, on-time, waveform characteristics, etc.). The communications device may be worn, such as a collar around the neck, or handheld.

In use, an implant may be configured to be implanted so that the electrodes contact or approximate the vagus nerve or a portion of the vagus nerve. In one variation the implant includes a cuff that at least partially surrounds the vagus (e.g., near the carotid region). The controller and/or signal generator (including any power source) may be formed as part of the cuff or may be connected to by a connector (e.g., wire).

In some variations the device may be non-invasive. For example, the device may be worn outside the body and may trigger stimulation of the vagus nerve from a site external to the body (e.g., the ear, neck, torso, etc.). A non-invasive device may include a mechanical device (e.g., configured to apply vibratory energy). In some variations the device is configured to apply ultrasound that may specifically target the vagus nerve and apply energy to activate the vagus nerve. In some variations, transcutaneous magnetic stimulation of the vagus nerve may be used.

In any of the variations described herein, the devices, system and methods may be configured to prevent desensitization of the signal in a way that would reduce or inhibit the modulation of demyelination and/or remyelination. For example in some variations, "over stimulation" of the vagus nerve, e.g., simulation at intensities that are too great or applied for too long, or outside of the frequency ranges described herein, may result in desensitization of the effect, thus further modulation may be limited or inhibited. Therefore, in some embodiments, the amplitude of stimulation may be restricted from exceeding (i.e., be less than) about 3 mA, 4 mA, or 5 mA, and/or the duty cycle may be restricted from exceeding about 1, 2, 3, 4, 5, 6, 7, 8, 9, 10, 15, 20, or 25%. In some embodiments, the amplitude is also at least 0.25 mA, 0.5 mA, 0.75 mA, or 1.0 mA.

The examples illustrated above may provide insight into the devices, systems and methods of use for stimulation of the vagus nerve to modulate demyelination and/or remyelination. These methods and devices may be used to treat any indication for which modulation of demyelination and/or remyelination would be beneficial. Non-limiting examples of indications include neurodegenerative and neuroinflammatory diseases such as multiple sclerosis (MS), Alzheimer's disease, Parkinson's disease, Huntington's disease, Amyotrophic lateral sclerosis (ALS), and Batten disease. Other examples include peripheral neuropathies, cranial neuropathies, and autonomic neuropathies. In general, these devices may offer alternative and in some ways superior treatment as compared to pharmacological interventions aimed at modulating demyelination and/or remyelination, and therefore may be used for any indication for which such pharmacological treatments are suggested or indicated. In some embodiments, the VNS treatments described herein can be used in conjunction with pharmacological treatments, particularly when the pharmacological treatment has a different mechanism of action than the VNS, which may lead to synergistic results.

Thus, the methods of modulating demyelination and/or remyelination as described herein may be used in conjunction with one or more pharmacological interventions, and particularly interventions that treat diseases associated with demyelination, neurodegeneration or neuroinflammation. For example, it may be beneficial to treat a subject receiving stimulation of the vagus nerve to modulate demyelination and/or remyelination by also providing agent such as intravenous corticosteroids (e.g., methylprednisolone), oral corticosteroids, interferons beta-1a and beta-1b, monoclonal antibodies (e.g., natalizumab, alemtuzumab, daclizumab and ocrelizumab), and immunomodulators (e.g., glatiramer acetate, mitoxantrone, fingolimod, teriflunomide, and dimethyl fumarate).

Thus, described herein are devices (VNS devices) for the treatment of neurodegenerative and/or neuroinflammatory disorders. Such devices are generally configured to apply low duty-cycle stimulation to the vagus nerve of a subject, as described in any of the variations (or sub-combinations) of these variations. In some embodiments, the patient is first diagnosed or identified with a neurodegenerative or neuroinflammatory disorder, particular a disorder characterized by demyelination or need for remyelination, before being implanted and treated with the VNS device.

In use, any of the methods described herein may include a step of monitoring for demyelination or demyelination -associated disorders, which may be determined through detection of a biomarker from blood and/or cerebrospinal fluid, and/or through medical imaging techniques such as MRI or CT scans. For example, as assay for an inflammatory cytokine (e.g., tumor necrosis factor) may be used to detect acute inflammatory episodes. Monitoring may be continuous or discrete (e.g., at one or more times, or time intervals). In addition or alternatively, biomarkers associated with multiple sclerosis or other neurodegenerative or neuroinflammatory diseases or neuropathies can be used for monitoring, depending on the disease being treated. See Housley, W. J., D. Pitt and D. A. Hafler (2015). "Biomarkers in multiple sclerosis." Clin Immunol 161(1): 51-58; and Katsavos, S. and M. Anagnostouli (2013). "Biomarkers in Multiple Sclerosis: An Up-to-Date Overview." Mult Scler Int 2013: 340508. For example, biomarkers found in MS serum and cerebrospinal fluid include markers of neurodegeneration including neurofilament and GFAP, the monocyte macrophage marker CD163, the glial activation marker YKL-40, the B cell chemoattractant CXCL13, miRNA and mRNA, myelin reactive t cells, Kir4.1 antibodies, osteopontin, and microbiome associated lipopeptides. Any of these biomarkers can be monitored and/or measured alone or in combination, and can be used as feedback to modulate VNS. Other biomarkers for treating MS patients in particular are listed in Table 1.

**Table 1. Biomarkers in Multiple Sclerosis**

| (A) Diagnostic biomarkers (criteria i, iv, v, and vi) | | |
|---|---|---|
| | | |
| (1) Genetic-immunogenetic | | |
| HLA-DRB1*1501 | +++ Risk for MS | See also B, E |
| DR3 and DR4 haplotypes | ++ **Risk for** MS | |
| HLA-DRB1*04 | ++ Risk for MS | |
| HLA-DRB 1*0401 | + Risk for high familial autoimmunity in MS patients | See also F |
| HLA-DQ1*0102 | + Risk for MS, in coexistence with HLA-DRB1*1501 | |
| HLA-DPB1*0501 | + Risk for opticospinal MS | |
| HLA-DPB1*0301 | + Risk for opticospinal MS | |
| IL2RA and IL7RA polymorphisms | + Risk for MS | |
| EVI5, CD58, KIAA0350, and RPL5 polymorphisms | +/- Risk for MS | |

| (2) Laboratorial | | |
|---|---|---|
| OCB IgG | +++ But with low specificity | See also E |
| KFLC | +++ But with low specificity | See also E |
| MRZ reaction | +++ Higher specificity than OCB IgG | See also E, F |
| Anti BRRF2, anti EBNA-1 | ++ | See also B, C |
| Anti MBP 48-70 and 85-170 | + | See also B, E |
| Anti MBP 43-68 and 146-170 | + Differential diagnosis with OND's | See also B, E |
| MBP/MOG conformational epitopes antibodies | + But low specificity | See also B, E, F |
| VEGF-A | + Lower CSF levels in all disease forms, but low specificity | See also D, E |
| Vitamin D | +++ Lower levels, higher risk for MS | See also C, F |
| TRECs | + Lower serum levels in all disease forms, but low specificity | See also B |
| CSF levels of lipocalin 2 | + Higher CSF levels in MS, but low specificity | See also F |
| AR | +++ Differential diagnosis of MS and NMO | See also C, E |
| NO and NO metabolites | + Higher CSF and serum levels in MS, but low specificity | See also C, E |
| NF-L | ++ Higher CSF levels in MS patients | See also C, F |
| NAA | +++ Differential diagnosis of RRMS and NMO | See also D, E |
| GFAP | +++ Differential diagnosis of MS and NMO | See also C, E |
| | + Differential diagnosis of MS and NMO | See also C, E |
| Nogo-A | ++ For MS forms with prominent neurodegenerative element | See also D |

| (3) Imaging | | |
|---|---|---|
| Contrast-enhanced T1 lesions | +++ | See also C |
| Hyperintense T2-weighted lesions | +++ | See also C, D, E |
| Corpus callosum DTI abnormalities | ++ Early diagnostic biomarker | See also E |
| MRS findings (glutamate/choline) | +++ | See also C, D, E |
| PET | ++ But still experimental | |
| EPs | +++ | See also C, D, E |
| Motor EPs | +++ Spinal cord syndrome at presentation | |
| VEMPs | +++ Brainstem dysfunction | |
| SSR | ++ Autonomic dysfunction assessment in MS patients | See also E |

| (B) Biomarkers of phenotypical expression (criteria ii, iv, v, and vi) | | |
|---|---|---|
| | | |
| (1) Genetic-immunogenetic | | |
| HLA-DRB1*1501 | +++ Early disease onset | See also A, E |
| HLA-DRB1*1501 | + Risk for cognitive decline | |
| HLA-DRB1*01 | ++ Protection against malignant disease form | |
| ApoE ε4 | ++ Greater risk for mental disorders | |

| (2) Laboratorial | | |
|---|---|---|
| OCB IgM against myelin lipids | +/- Aggressive disease course | See also E |
| EBV antibodies | + Early disease onset | See also A, C |
| Anti-MBP | +++ ADEM-like onset in childhood MS | See also A, E |
| Anti-MOG | +++ Childhood MS, ADEM, isolated optic neuritis, anti-AQP4 (-) NMO | See also A, E, F |
| rMOG index | +++ Progressive disease forms | |
| IL-6 serum levels | +++ Age at onset | See also C |
| TRECs | ++ Lower levels PPMS | See also A |
| Amyloid- (1-42) | ++ Lower levels, higher risk for mental disorders | |

| (3) Imaging | | |
|---|---|---|
| UCCA atrophy | +++ Progressive disease forms | See also E |
| NAGM DTI abnormalities | +++ Progressive disease forms | |
| | | |

| (C) Biomarkers of demyelination-neuroinflammation-relapse (criteria i, ii, iii, iv, v, and vi) | | |
|---|---|---|
| | | |

| (1) Genetic-immunogenetic | | |
|---|---|---|
| TOB1 | +++ Underexpression, higher Th1 and Th17 percentage | See also E |

| (2) Laboratorial | | |
|---|---|---|
| EBV antibodies | + Higher inflammatory activity | See also A, B |
| CXCL13 | ++ Mobilizes B-cells, T-helper cells | |
| CXCL12 | +/- Neuroprotection against inflammation in | |
| | EAE/ experimental | |
| IFN-/TNF-a | +++ Th1 immune response | |
| IL-1 levels imbalance | + Triggering factor for neuroinflammation | |
| IL-6 | +++ B-cell and T-cell immunity link, Th17 immune response triggering factor | See also B |
| | ++ Correlation with relapse frequency in female MS patients | |
| IL-10 -592 position polymorphisms | ++ Regulation of CNS autoimmunity | |
| IL-15 | ++ BBB disruption, enhanced CD8(+) T cytotoxicity | |
| IL-33 | + Increase in IFN-γ and IL-17 in mice EAE | |
| sICAM-1 | ++ Higher levels, higher inflammatory activity | See also F |
| | +++ Higher levels in NMO than MSmarker of BBB disruption | |
| sVCAM-1 | +++ Higher levels in NMO than MS-marker of BBB disruption | See also F |
| Laminin 411 | ++ TH-17 enhancement | |
| 4 Integrin | ++ Correlation with gadolinium-enhanced lesions during CIS | See also E, F |
| Osteopontin | ++ Serum and CSF elevation during relapse | |
| Fetuin-A | +++ Overexpression in active demyelinating lesions | See also F |
| Vitamin D | +++ High levels, anti-inflammatory role-lower radiological disease activity | See also A, F |
| CSF mature B-cells/plasma-blasts | ++ Bigger accumulation, higher inflammatory activity | |
| CXCR3 | ++ Helps T-cells to enter the brain | |
| CX(3)CR1 | ++ CD4(+)CD28(-) cytotoxic cells biomarker | |
| CSF CCR2(+)CCR5(+) T cells | +++ Increase during MS relapseosteopontin enhancement | |
| CD56 Bright NK | ++ Remission phase | |
| AR | +++ Biomarker of BBB disruption | See also A, E |
| MMP-9 | ++ Higher CSF levels during relapse | |
| Ninjurin-1 | ++ Upregulation in active demyelinating lesions | |
| MBP and fragments | +++ Higher CSF levels during relapse | See also F |
| B-Crystalline | +++ Over-expression in active demyelinating lesions | |
| NO and metabolites | ++ | See also A, E |
| 7-Ketocholesterol | ++ | |
| Glutamate | +++ Higher levels in active demyelinating lesions | |
| Cystine/glutamate antiporter | + Over-expression in active demyelinating lesions | |
| NF-L | +++ Higher CSF levels, especially the 3rd week after relapse onset | See also A, F |
| GFAP | ++ Higher levels during relapse | See also A, E |
| S100B | +/- Higher CSF levels during MS/NMO relapse | See also A, E |
| N-CAM | + CSF elevation at remission onset | |
| BDNF | ++ Lower levels inhibit demyelination and axonal loss | See also D, E, F |

| (3) Imaging | | |
|---|---|---|
| Contrast-enhanced T1 lesions | +++ Active lesions | See also A |
| Hyperintense T2-weighted lesions | ++ Combination of different mechanisms | See also A, D, E |
| MTR decrease | + Demyelination and axonal loss combined | See also D |
| DTI abnormalities | ++ Combination of different mechanisms | See also D, E |
| MRS findings (especially changes in glutamate and choline) | +++ Active lesions | See also A, D, E |
| DTS | ++ Promising but still experimental | See also D |
| EP's delayed conduction | ++ Demyelination biomarker | See also A, D, E |
| | | |

| (D) Biomarkers of axonal loss-neurodegeneration (criteria i, iv, v, and vi) | | |
|---|---|---|
| | | |

| (1) Laboratorial | | |
|---|---|---|
| VEGF-A | ++ Lower levels, higher risk for neurodegeneration | See also A, E |
| 14-3-3 | +/- Axonal loss | |
| NAA | +++ Axonal loss | See also |
| | | A, E |
| BDNF | ++ Lower levels inhibit demyelination and axonal loss | See also C, E, F |
| Nogo-A | +++ Higher CSF levels, failure in axonal repair | See also A |

| (2) Imaging | | |
|---|---|---|
| RNFL thinning | +++ Axonal loss in the optic nerve | See also E, F |
| Hyperintense T2-weighted lesions | ++ Combination of different mechanisms | See also A, C, E |
| Black holes | +++ Axonal loss | See also E |
| MTR decrease | ++ Demyelination and axonal loss combined | See also C |
| DTI abnormalities | ++ Combination of different mechanisms | See also C, E |
| MRS findings (especially NAA) | ++ | See also A, C, E |
| DTS | +++ Promising but still not widely accessible | See also C |
| Visual and motor EPs | ++ | See also A, C, D |
| | | |

| (E) Prognostic biomarkers-biomarkers of disability progression (criteria ii, iv, v, vi, and viii) | | |
|---|---|---|
| | | |

| (1) Genetic-immunogenetic | | |
|---|---|---|
| HLA-DRB1* 1501 | +/- Early progression from RRMS to SPMS | See also A, B |
| HLA-DRB1*1501 | + Worst brain atrophy measures | |
| HLA-DQB1*0301 | + Worst brain atrophy measures | |
| HLA-DQB 1*0602 | + Worst whole and gray matter atrophy measures | |
| TOB1 | +++ Early conversion from CIS to CDMS | See also C |

| (2) Laboratorial | | |
|---|---|---|
| OCB IgG | +++ Conversion from CIS to CDMS | See also A |
| KFLC | +++ Conversion from CIS to CDMS | See also A |
| OCB IgM | +/- Bad prognostic biomarker | See also B |
| MRZ reaction | +++ Conversion from CIS to CDMS | See also A, F |
| Anti-MBP | +/- Conversion from CIS to CDMS | See also A, B |
| Anti-MOG | +/- Conversion from CIS to CDMS | See also A, B, F |
| AR | ++ Marker of clinical severity in NMO | See also A, C |
| VEGF-A | ++ Lower levels, progression from RRMS to SPMS | See also A, D |
| NO and NO metabolites | ++ Higher CSF levels, longer relapses/higher disability progression rates | See also A, C |
| NF-H | +++ Higher CSF levels, progressive forms/bad prognostic biomarker | |
| NF-H and tau | +++ Combined high CSF levels, conversion from CIS to CDMS | |
| Tubulin/actin | ++ Higher CSF levels, progressive forms/worst disability scores | |
| NAA | +++ Lower CSF levels, progressive forms/worst disability scores | See also A, D |
| GFAP | ++ Higher CSF levels, progressive MS forms/worst disability scores | See also A,C |
| | +++ Disability progression in NMO | |
| S100B | + Disability progression in NMO | See also A,C |
| BDNF | ++ Lower CSF levels in SPMS patients | See also C, D, F |

| Unblocked α4 integrin | + Prognostic factor of risk for PML | See also C, F |
|---|---|---|
| (3) Imaging | | |
| RNFL thinning | + Correlation with brain atrophy measures and disease progression | See also D, F |
| Hyperintense T2-weighted lesions | +/- | See also A, C, D |
| Black holes | +/- | See also D |
| Whole brain atrophy measures | ++ Worsening rates at MS onset, prognostic biomarker of disability after 8 years | |
| Gray matter atrophy measures | +++ Higher worsening rates, progressive forms/early CIS conversion to RRMS | |
| UCCA atrophy | ++ Progressive forms, good correlation with | See also |
| | EDSS, bad prognostic in RRMS | B |
| DTI abnormalities | +++ Early prognostic biomarker of relapse | See also C, D |
| Corpus callosum DTI abnormalities | +++ Bad prognostic biomarker | See also A |
| Spinal cord DTI abnormalities | +++ Good correlation with EDSS scores | |
| Early MRS abnormalities | ++ Bad prognostic biomarker | See also A, C, D |
| Combined EPs | +++ Good prognostic biomarker, especially for benign disease forms | See also A, C, D |
| SSR | ++ Correlation with higher EDSS scores | See also A |
| | | |

| (F) Biomarkers of therapeutical response (criteria i, iv, v, vi, and vii) | | |
|---|---|---|
| | | |

| (1) Genetic-immunogenetic | | |
|---|---|---|
| HLA-DRB1*0401, 0408, 1601 | +++ Higher risk for developing neutralizing antibodies against IFN-B | See also A |

| (2) Laboratorial | | |
|---|---|---|
| MRZ reaction | ++ B-cell immunity targeted therapy | See also A, E |
| Anti-MOG | ++ B-cell immunity targeted therapy | See also A, B, E |
| Fetuin-A | +++ Decreased CSF levels in Natalizumab responders | See also C |
| MBP | +++ Decrease in CSF levels in methylprednizolone responders | See also C |
| CSF lipocalin 2 | ++ Decreased CSF levels in Natalizumab responders | See also A |
| Unblocked α4 integrin | +++ Therapeutical response to Natalizumab | See also C, E |
| NF-L | +++ Normalized CSF levels in Natalizumab responders | See also A, C |
| BDNF | +++ CSF elevation in Glatiramer Acetate responders | See also C, D, E |
| TRAIL | ++ Serum levels good predictors of response in IFN-B | |
| MxA | ++ Serum levels good predictors of response in IFN-B | |
| sVCAM | ++ CSF alterations in IFN-B responders | See also C |
| Th17 immune profil | +/- Immune response exacerbation by IFN-B | |
| Vitamin D | +++ Increased levels in IFN-B responders | See also A, C |
| sICAM-1 | + Lower levels in Cladribine responders | See also C |
| sE-Selectin | + Lower levels in Cladribine responders | |

| (3) Imaging | | |
|---|---|---|
| RNFL | +++ Biomarker of therapeutical efficacy for several agents | See also D, E |
| Classification of biomarkers. +++ very strong correlation, ++ strong correlation, + modest correlation, and +/- controversial correlation. Criteria used for classification., (i) Biological rationale; (ii) clinical rationale; (iii) predictability of disease initiation, reactivation or progression, or of disease differentiation; (iv) sensitivity and specificity; (v) reproducibility; (vi) practicality; (vii) correlation with therapeutical outcome; (viii) correlation with prognosis and disability. Biomarkers of more than one category are indicated in the third column. | | |

The information described herein for the first time shows that stimulation of the vagus nerve modulates demyelination and/or remyelination and/or leaking through the blood-brain barrier. The examples provided herein are not intended to be comprehensive, but merely illustrate and embody certain variations of the invention. It is to be noted that the scope of the present invention is defined by the appended claims.

### Example 1

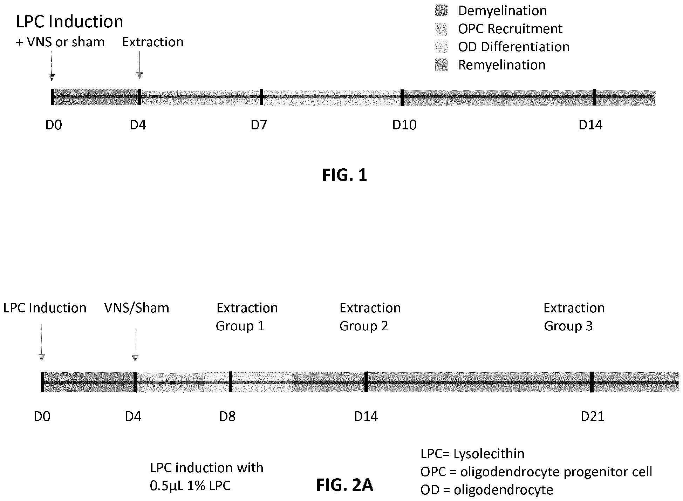

To study the effect of VNS on neurodegeneration and neuroinflammation, a lysolecithin (LPC) - induced MS model can be used. Lysolecithin is a bioactive proinflammatory lipid that is a detergent-like membrane solubilizing agent. A 1% solution of LPC can induce local demyelinating lesions when injected into the white matter of the spinal cord. Four distinct epochs occur over 14 days post-injection: (1) demyelination; (2) oligodendrocyte progenitor cell (OPC) recruitment; (3) differentiation; and (4) remyelination. FIG. 1 illustrates a typical example of the 4 epochs, where demyelination occurs from about days 0-3, OPC recruitment occurs from about days 3-7, OPC differentiation occurs from about days 7-10, and remyelination occurs from about days 10-14.

To induce a self-limited demyelinating lesion, spinal cords of female BALB/c mice were injected between T3-T5 with 1% LPC (0.5 µL at 0.25 µL/min). The procedure to inject the mice with LPC was as follows. The mouse was anesthetized and stabilized into a stereotaxic frame. A midline incision was made between the scapulae. The underlying fat pads were bluntly separated and the spinous process of the T2 vertebra was identified and a laminectomy was performed. A syringe was advanced to 0.3 mm into the spinal cord and 0.5 uL of LPC was injected at a rate of 0.250 uL/min for 2 min. The muscle and adipose tissue were sutured and the skin was closed with surgical staples

VNS was performed as previously described (Olofsson, Levine, et al. 2015. Bioelectronic Medicine: 37-42) on Day 0 or Day 4 post-induction with LPC. More specifically, to study the effect of VNS on demyelination, VNS (0.75-1 mA, 250 µS pulse, 10 Hz) or sham VNS (0 mA) was performed immediately following LPC administration, and the mice were euthanized on the day of expected peak lesion volume (day 4 post-induction; J Neurocytol 24(10): 775-81). The demyelination experimental protocol is summarized in FIG. 2A.

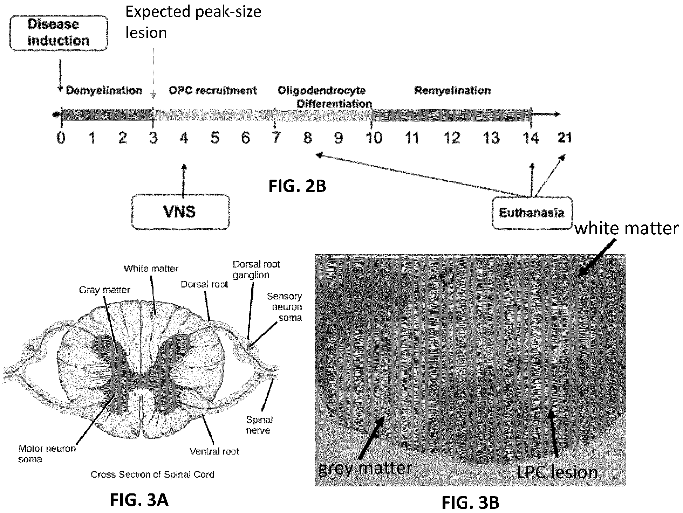

Spinal cord lesion volumes/areas were quantified by myelin loss as assessed from luxol blue-stained, 15 µm serial sections. FIG. 3A shows an illustration of a typical cross-section of the spinal cord, and FIG. 3B shows a luxol blue stained cross-section of the spinal cord with a LPC induced lesion in the anterior funiculus of the white matter 5 days post-LPC injection. To study the effect of VNS on remyelination, VNS or sham VNS treatments was performed 4 days post-induction, mice were euthanized on days 8, 14, or 21 post-induction, and nerves were processed as above. The remyelination experimental protocol is summarized in FIG. 2B. Mean lesion volumes between groups were compared by t-test.

Results: The demyelination protocol illustrated in FIG. 2A showed that VNS inhibited demyelinated lesion progression compared to sham. On day 4 post-induction, the mice were euthanized and the spinal cord around the LPC injection site was sectioned and stained with luxol blue. As shown in FIGS. 4A-4D, the mean lesion volume in the VNS group (0.75 mA) was significantly lower than in the sham group (VNS = 0.03 mm3 ± 0.006, n=5, vs. Sham= 0.09 mm3 ± 0.009, n=4, p = 0.0023). VNS at 0.25 mA resulted in a mean lesion volume similar to sham VNS.

The remyelination protocol illustrated in FIG. 2B showed that remyelination occurred at a significantly accelerated rate in the VNS group. As shown in FIGS. 5A-5G, on day 8 post-induction, mean lesion volume in the VNS group was reduced to 0.02 mm3 ± 0.01, n=4. On day 14 post-induction, mean lesion volume in the VNS group was significantly lower than in the sham group (VNS = 0.0002 mm3 ± 0.007, n=12, vs. Sham= 0.03 mm3 ± 0.01, n=6, p = 0.0007). On day 14, 11 out of 12 VNS animals had no detectable lesion. By Day 21, the mean lesion volume in the sham group was 0.01 mm3 ± 0.006, n=3. FIG. 5A shows that the area under the curve (AUC) between days 4 and 21 is reduced by about 65 percent with vagus nerve stimulation.

Conclusions: VNS reduced demyelination and accelerated remyelination, demonstrating a robust effect after a single dose in this model. Repeated stimulation of the vagus nerve with an implanted nerve stimulator may further reduce the rate of demyelination and/or further accelerate remyelination. This will be tested in an experimental autoimmune encephalomyelitis model to further assess the potential of VNS to treat MS.

### Example 2

Another study was performed to determine the effect of VNS on vessel leakiness 24 hours post-induction and stimulation. A lesion was induced as described above using LPC injection and VNS was performed immediately following induction. At 24h, 0.15 mL of 1% Evans blue dye was injected intravenously through retro-orbital injection under anesthesia for 1 hr., as shown in FIG. 6A. One hour later, the animals were euthanized via cervical dislocation. Measurement of extravasation in the spinal cord (SC) was determined by extracting the SC and weighing the SC wet. The SC was then dried for 24h at 56°C and weighed dry. The Evans blue dye was extracted with a formamide solvent for 48h at 56°C incubation. The supernatant was measured spectroscopically at 620 nm and the quantity of Evans blue dye was determined by interpolation from a reference curve. The quantity of Evans blue dye was normalized to the dry weight of the SC. As shown in FIG. 6B, less Evans blue dye was extracted from the spinal cord from the mice that received VNS, which provides evidence that VNS reduces vessel leakiness 24 hours post-induction and stimulation. In addition, the amount of Evans blue dye extracted from the mice that received VNS was similar to the amount of Evans blue dye extracted from naive mice (no LPC induced lesion).

Leakiness in the blood brain barrier may allow immune cells and inflammatory cytokines and chemokines to pass through and contribute to continued inflammation in the brain and/or spinal cord. Therefore, VNS may reduce vessel leakiness around the central nervous system (CNS), thereby reducing the recruitment of proinflammatory cells such as lymphocytes (e.g., T-cells) and macrophages to the brain and spinal cord, thereby reducing the inflammation in the CNS and reducing the amount demyelination that results from an inflammatory attack by the immune system.

### Example 3

In general, the apparatuses and methods described for VNS therapy may also be used to prevent or treat increased leakiness of the blood-brain barrier, as illustrate in FIG. 6B.

Methods: 1% LPC was injected into the spinal cord white matter of BALB/c mice. For the first intervention time point, VNS therapy or sham VNS was performed immediately after injection. 24 hours later, mice (VNS, sham VNS, and naive (no-LPC)) are injected with 1% Evans blue dye which binds to the albumin in blood and is left to circulate for 1 hour. Spinal cords are then harvested, dried for 24 hours in pre-weighed tubes at 60°C. Dried tissues are then incubated in formamide for 48 hours. Supernatant is then extracted from the tubes and read spectroscopically at 620 nm. For the second intervention time point, VNS therapy or sham VNS therapy occurs on day 4 post-LPC induction. On day 5 post-LPC induction, Evans blue extravasation is performed the same way as described for demyelination experiment. Evans blue concentration is compared (ng/mg of tissue) and normalized to naive animals.

Results: LPC increased blood-spinal cord leakiness. VNS therapy significantly reduced Evans blue extravasation into the spinal cord compared to sham (81% decrease) 24 hours post-LPC induction (FIG. 6B). In addition, VNS therapy on day 4 post-LPC significantly reduced Evans blue extravasation on day 5 compared to sham (52% decrease).

Conclusion: VNS therapy increases the integrity of the blood-spinal cord barrier and subsequently reduces the extravasation of protein/Evans blue and other circulating species, including antibodies, DAMPS/PAMPS, and immunocytes into the central nervous system.

### Example 4

Another experiment was performed to determine whether the effect of VNS on demyelination was α7 nicotinic acetylcholine receptor (nAChR) dependent. Two mice strains were used in the study. One mice strain is the C57 Black subtype 6 (C57BL/6), which is a common wild type strain that expresses α7 receptors and are denoted as α7+/+. The second mice strain is an α7 knockout strain of the C57BL/6 strain, which lacks the α7 receptor and are denoted as α7-/-. Each of the mice strains were given LPC injections in sham (no VNS) and VNS groups. Tissue extraction was performed 4 days post-injection. The procedure was essentially identical to the Balb/c mice demyelination experiments described above in Example 1.

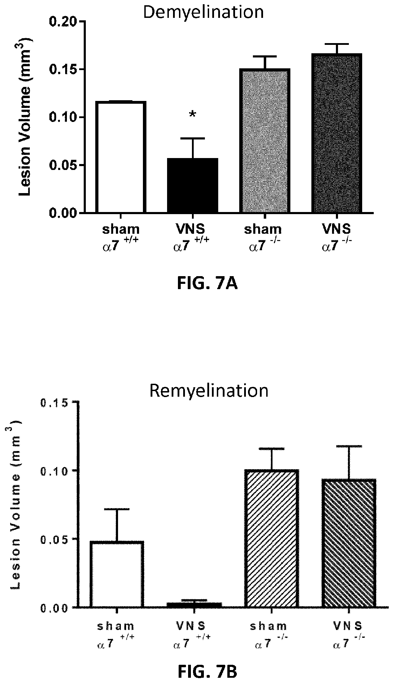

As shown in FIG. 7A, the protective effects of VNS on demyelination is α7 nAChR-dependent. VNS treatment on mice with the α7 nAChR showed a reduced lesion volume when compared with sham, while VNS treatment on mice without the α7 nAChR showed no reduction in lesion volume when compared with sham. Similarly, the remyelination effect of VNS treatment may be α7 nAChR dependent, as shown in FIG. 7B. In this example, the effect of VNS treatment on remyelination in the presence (+/+) and absence (-/-) of the α7 nAChR due to either sham (no VNS treatment) or VNS treatment were examined, showing a substantial decrease in lesion volume, the maker for re-myelination following induction of a demyelination event (e.g., application of LPC.

In FIGS. 7A-7B, 1% LPC was injected into the spinal cord white matter of α7 nAChR knockout mice and C57BL/6 (wildtype) mice. For demyelination experiment, VNS treatment or sham VNS treatment, tissue collection, processing, and analysis are all the same as mentioned above for FIG. 1A. For remyelination, VNS and sham VNS intervention occurs the same as experiment described for Figure 4B. Spinal cords are harvested only on day 8 post- LPC induction. Processing and analysis performed are the same as described for Figures 4A-4B.

Result: VNS therapy decreased demyelination in wildtype C57BL/6 mice. VNS therapy did not decrease demyelination in α7 KO animals (FIG. 7A). VNS therapy increased remyelination in wildtype animals, but did not increase remyelination in the knockouts (Figure 7B). Thus, the effects of VNS on demyelination and remyelination are α7-dependent.

### Example 5

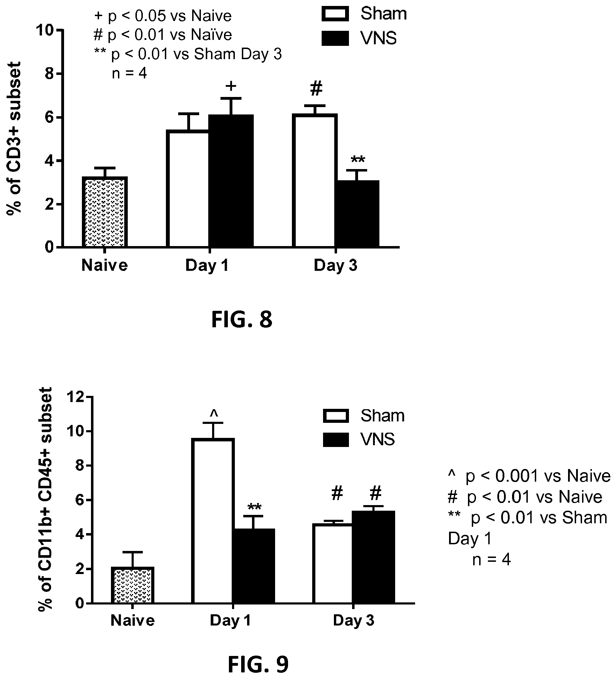

In general, the apparatuses and methods described for VNS therapy may also be used to prevent or treat increased immunocyte homing to the central nervous system, as illustrate in FIG. 8 and 9.

In FIGS. 8 and 9, CD3+ T cell infiltration through a model for the blood-brain barrier is significantly decreased in the VNS treatment group. As shown in FIG. 8, the CD3+ T cell infiltration through the model of the blood-brain barrier on Day 3 post-LPC induction compared to Sham group is reduced by 50%. In. FIG. 9, the macrophage infiltration is significantly decreased 24 hours post-LPC induction in the VNS treatment group compared to the Sham (no VNS treatment) group by 55%.

Methods: Surgical procedures and VNS/sham VNS treatments remain the same from FIG. 4A. Spinal cords from VNS therapy, sham, and naive mice are harvested on days 1 or 3 post-LPC induction. Tissue is then digested in enzymatic cocktail for 20 minutes at 37°C followed by trituration and filtering through a 100µM mesh screen. Single cell suspension is then put through a density gradient to remove myelin debris from glia cells and immune cells. Once isolated, cells are blocked in FACS buffer and CD32/CD19 for a half hour to prevent unspecific antibody staining. Cells are counted and checked for viability via hemocytometer. Cells are then placed in tubes, stained for either T cells (CD3+) or macrophages (CD11b+, CD45hi) and then analyzed via flow cytometer. Populations of cells are quantified using FlowJo program.

Result: LPC increased CD3+ T cell and macrophage infiltration in the spinal cord compared to naive tissue (FIGS. 8 and 9). There was a significant reduction in CD3+ T cell infiltration on day 3 post-LPC induction in VNS therapy treated animals compared to sham (50% reduction) (FIG. 9). In addition, VNS therapy resulted in a significant decrease in macrophage infiltration compared to sham 1 day post-LPC induction (55% reduction) (FIG. 9). Thus VNS significantly reduces the infiltration of peripheral immunocytes into the CNS in this lysolecithin-induced MS model.

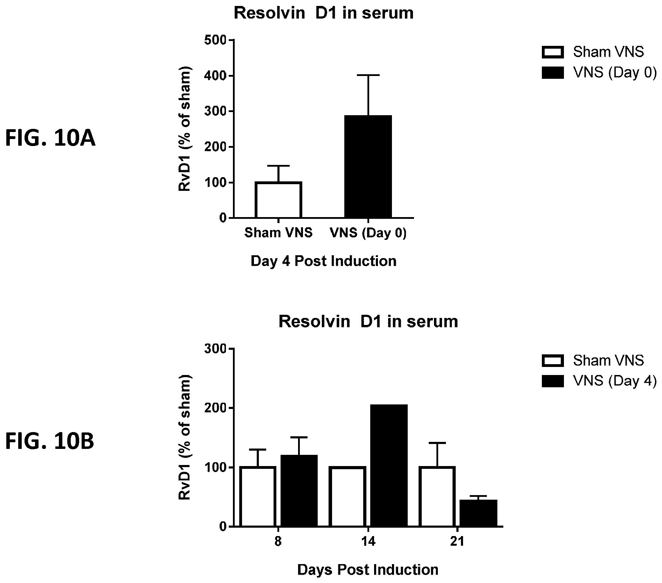

As shown in FIGS. 10A-10B, VNS therapy also increased re-myelination following a decrease in myelination. During spinal cord extractions for all prior experiments performed (see examples 1-4, above), blood was collected via cardiac puncture as well. Blood was centrifuged at 8,000 xg for 5 minutes, the serum was collected and stored at -80°C. Using a Resolvin D1 ELISA kit, levels of RvD1 were measured spectroscopically from the serum of VNS and sham VNS mice for the demyelination (D4 harvest) and remyelination (D8, D14, and D21 harvests) experiments. Levels of RvD1 are analyzed (pg/mL) and represented as a percent of sham by day.

Result: as showing FIG. 10A, VNS therapy on day 0 (LPC-induction) increased serum levels of RvD1 on day 4. As shown in FIG. 10B, VNS on day 4 post-LPC induction also increased RvD1 in the serum levels of RvD1 with the highest concentration occurring on day 14 post-LPC induction. RvD1 levels in VNS serum were decreased as compared to sham at 21 days post-LPC induction, likely due to earlier resolution in the VNS group.

Thus, VNS therapy increases the pro-resolving lipid mediator RvD1 in serum which may contribute to the increased speed in resolution time of LPC-induced lesions compared to sham.

### EXAMPLE: System

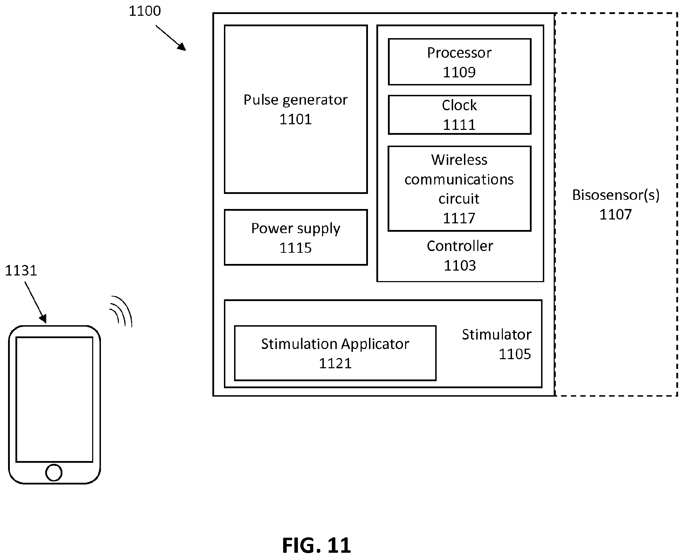

FIG. 11 schematically illustrates one example of a system 1100 for treating demyelination (e.g., for treating MS, or any other demyelinating disorders). In some variations the system for reducing demyelination and/or increase remyelination by stimulation of a vagus nerve includes a controller 1103, a stimulator 1105, and a pulse generator 1101. The pulse generator and stimulator may be connected to and controlled by the controller. In some variations all or some of the system may be implanted into the patient's body. All or some of the components of the apparatus may be enclosed by a housing (e.g., an implant housing). In general, the systems may also include one or more biosensor 1107 configured to detect one or more biomarkers. The biosensor may be coupled with the rest of the system (e.g., implant) or it may be separate and may communicate via a wired or wireless connection. For example the biosensor may be implanted into the body so as to sample blood, spinal fluid, or the like; in some variations the biosensor is external to the body and may be single use or configured for limited-reuse. In some variations the biosensor may include a sensor for determining a patient's physical condition (e.g., temperature, nerve conduction, etc.). In some variations the biosensor may be an immunochemical sensor configured to detect binding of one or more analytes and/or to provide a concentration.

The stimulator may be configured to apply stimulation to the vagus nerve. A stimulator maybe configured for electrical stimulation, mechanical stimulation, or both. For example, the stimulator may include or be coupled with the pulse generator 1101 (e.g., waveform and/or pulse generator, oscillator, etc.). The stimulator may include one or more stimulation applicators 1121 (e.g., one or more electrodes, mechanical transducers, etc.) for contact with the tissue, including the vagus nerve.

Any of the apparatuses may also include one or more power supplies 1115, and/or power regulation circuit, etc.

The controller is functionally coupled to the one or more biosensor (e.g., receiving data from the biosensor(s)) and controls the stimulator and may be configured to apply stimulation to the vagus nerve from the stimulator sufficient to reduce demyelination and/or increase remyelination of nerves within the patient when the biosensor detects a biomarker indicative of active demyelination.

For example, a system may include an implant comprising a stimulator (e.g., a waveform and/or pulse generator, an oscillator, a power supply and/or power regulation circuit, etc.), a stimulation applicator (e.g., one or more electrodes, mechanical transducers, etc.), and a controller. The controller may be configured as a microcontroller and may be in electrical communication with the stimulator so as to control operation of the stimulator. The controller may include one or more processors, a memory and/or a timer. The stimulator and/or controller may be in electrical communication, one or more stimulation applicators. In some variations the controller may include or be in communication with wireless communications circuitry 1117 for wirelessly communicating with one or more remote processors 1131. The remote processor may be a hand-held device (e.g., smartphone, wearable electronics, etc.). The controller may optionally be in communication with one or more biosensors that may be included with the implant or may be remote from the implant (e.g., may be wearable, single-use, etc.). In some variations the biosensors are wirelessly connected to the apparatus.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as defined by the appended claims. Therefore, the foregoing description is provided primarily for exemplary purposes and should not be interpreted to limit the scope of the invention as it is set forth in the claims.

## Claims

1. A system for reducing demyelination and increasing remyelination of nerves by stimulation of a vagus nerve, the system comprising:
a biosensor configured to detect one or more biomarkers for active demyelination;
a stimulator configured to apply stimulation to the vagus nerve; and
a controller coupled to the biosensor and the stimulator and configured to apply electrical stimulation of between about 0.25 mA and about 5 mA at a low-duty cycle of less than 10 percent and with a pulse width of between 50 µs and 500 µs to the vagus nerve from the stimulator sufficient to reduce demyelination and increase remyelination of nerves within the patient when the biosensor detects a biomarker indicative of active demyelination.

2. The system of claim 1, wherein the biosensor is configured to detect a marker from the patient's blood and/or cerebrospinal fluid.

3. The system of claim 1, wherein the biosensor is configured to detect temperature.

4. The system of claim 1, wherein the system is configured to be implanted.

5. The system of claim 1, wherein the controller is configured to apply the low duty-cycle electrical stimulation, followed by an off-time of at least 10 minutes.

6. The system of claim 1, wherein the controller is configured to apply the low duty-cycle electrical stimulation for less than about 2 minutes.

7. The system of claim 1, wherein the controller is configured to apply the low duty-cycle electrical stimulation of between about 0.25 mA and about 5 mA to the vagus nerve for less than about 2 minutes, followed by an off-time of at least 10 minutes.

8. The system of claim 1, wherein the stimulator comprises an electrode configured to apply electrical energy to the vagus nerve.

9. The system of claim 1, further comprising a nerve cuff configured to secure the stimulator to the vagus nerve.

10. The system of claim 1, wherein the system is configured to continuously monitor the patient for the one or more biomarkers.

11. The system of any preceding claims, wherein at least one of the one or more biomarkers is selected from a group comprising: neurofilament, glial fibrillary acidic protein, the monocyte macrophage marker CD163, the glial activation marker YKL-40, the B cell chemoattractant CXCL13, miRNA, mRNA, myelin reactive t cells, Kir4.1 antibodies, osteopontin, and microbiome associated lipopeptides.

## Patentansprüche

1. System zur Verringerung der Demyelinisierung und Erhöhung der Remyelinisierung von Nerven durch Stimulation eines Vagusnervs, das System umfassend:
einen Biosensor, der konfiguriert ist, um einen oder mehrere Biomarker für aktive Demyelinisierung zu erkennen;
einen Stimulator, der konfiguriert ist, um den Vagusnerv zu stimulieren; und
eine Steuerung, die mit dem Biosensor und dem Stimulator gekoppelt und konfiguriert ist, um eine elektrische Stimulation zwischen etwa 0,25 mA und etwa 5 mA bei einem niedrigen Arbeitszyklus von weniger als 10 Prozent und mit einer Impulsbreite zwischen 50 µs und 500 µs vom Stimulator auf den Vagusnerv anzuwenden, die ausreicht, um die Demyelinisierung zu reduzieren und die Remyelinisierung von Nerven innerhalb des Patienten zu erhöhen, wenn der Biosensor einen Biomarker detektiert, der eine aktive Demyelinisierung angibt.

2. System nach Anspruch 1, wobei der Biosensor konfiguriert ist, um einen Marker aus dem Blut und/oder dem zerebrospinalen Fluid des Patienten zu erkennen.

3. System nach Anspruch 1, wobei der Biosensor konfiguriert ist, um die Temperatur zu erkennen.

4. System nach Anspruch 1, wobei das System konfiguriert ist, um implantiert zu werden.

5. System nach Anspruch 1, wobei die Steuerung konfiguriert ist, um die elektrische Stimulation mit niedrigem Arbeitszyklus anzuwenden, gefolgt von einer Auszeit von mindestens 10 Minuten.

6. System nach Anspruch 1, wobei die Steuerung konfiguriert ist, um die elektrische Stimulation mit niedrigem Arbeitszyklus für weniger als etwa 2 Minuten anzuwenden.

7. System nach Anspruch 1, wobei die Steuerung konfiguriert ist, um die elektrische Stimulation mit niedrigem Arbeitszyklus zwischen etwa 0,25 mA und etwa 5 mA für weniger als etwa 2 Minuten auf den Vagusnerv anzuwenden, gefolgt von einer Auszeit von mindestens 10 Minuten.

8. System nach Anspruch 1, wobei der Stimulator eine Elektrode umfasst, die konfiguriert ist, um elektrische Energie an den Vagusnerv anzuwenden.

9. System nach Anspruch 1, ferner umfassend eine Nervenmanschette, die konfiguriert ist, um den Stimulator am Vagusnerv zu befestigen.

10. System nach Anspruch 1, wobei das System konfiguriert ist, um den Patienten kontinuierlich auf einen oder mehrere Biomarker zu überwachen.

11. System nach einem der vorstehenden Ansprüche, wobei mindestens einer der einen oder mehreren Biomarker aus einer Gruppe ausgewählt ist, umfassend: Neurofilament, gliales fibrilläres saures Protein, den Monozyten-Makrophagen-Marker CD163, den glialen Aktivierungsmarker YKL-40, den B-Zell-Chemoattraktor CXCL13, miRNA, mRNA, myelinreaktive T-Zellen, Kir4.1-Antikörper, Osteopontin und Mikrobiom-assoziierte Lipopeptide.

## Revendications

1. Système permettant de réduire la démyélinisation et d'augmenter la remyélinisation des nerfs par stimulation d'un nerf vague, le système comprenant :
un biocapteur configuré pour détecter un ou plusieurs biomarqueurs de démyélinisation active ;
un stimulateur configuré pour appliquer une stimulation au nerf vague ; et
un dispositif de commande couplé au biocapteur et au stimulateur et configuré pour appliquer une stimulation électrique comprise entre environ 0,25 mA et environ 5 mA à un faible rapport cyclique inférieur à 10 % et avec une largeur d'impulsion comprise entre 50 µs et 500 µs au nerf vague à partir du stimulateur suffisante pour réduire la démyélinisation et augmenter la remyélinisation des nerfs chez le patient lorsque le biocapteur détecte un biomarqueur indicatif d'une démyélinisation active.

2. Système selon la revendication 1, dans lequel le biocapteur est configuré pour détecter un marqueur provenant du sang et/ou du liquide céphalo-rachidien du patient.

3. Système selon la revendication 1, dans lequel le biocapteur est configuré pour détecter la température.

4. Système selon la revendication 1, dans lequel le système est configuré pour être implanté.

5. Système selon la revendication 1, dans lequel le dispositif de commande est configuré pour appliquer la stimulation électrique à faible rapport cyclique, suivie d'un temps d'arrêt d'au moins 10 minutes.

6. Système selon la revendication 1, dans lequel le dispositif de commande est configuré pour appliquer la stimulation électrique à faible rapport cyclique pendant moins de 2 minutes environ.

7. Système selon la revendication 1, dans lequel le dispositif de commande est configuré pour appliquer la stimulation électrique à faible rapport cyclique comprise entre environ 0,25 mA et environ 5 mA au nerf vague pendant moins de 2 minutes environ, suivie d'un temps d'arrêt d'au moins 10 minutes.

8. Système selon la revendication 1, dans lequel le stimulateur comprend une électrode configurée pour appliquer de l'énergie électrique au nerf vague.

9. Système selon la revendication 1, comprenant également un manchon de nerf configuré pour fixer le stimulateur au nerf vague.

10. Système selon la revendication 1, dans lequel le système est configuré pour surveiller en continu le patient pour les un ou plusieurs biomarqueurs.

11. Système selon l'une quelconque des revendications précédentes, dans lequel au moins un des un ou plusieurs biomarqueurs est choisi dans un groupe composé de : neurofilament, protéine acide fibrillaire gliale, le marqueur monocyte-macrophage CD163, le marqueur d'activation gliale YKL-40, le chimioattractant des cellules B CXCL13, miARN, mARN, cellules t réactives à la myéline, anticorps Kir4.1, ostéopontine, et lipopeptides associés au microbiome.
